(19) Europäisches Patentamt / European Patent Office / Office européen des brevets

(11) **EP 3 285 706 B1**

(12) **EUROPEAN PATENT SPECIFICATION**

(45) Date of publication and mention of the grant of the patent:
**21.08.2019   Bulletin 2019/34**

(21) Application number: **16716428.4**

(22) Date of filing: **05.04.2016**

(51) Int Cl.:
*A61F 13/514* (2006.01)

(86) International application number:
**PCT/US2016/026027**

(87) International publication number:
**WO 2016/178768 (10.11.2016 Gazette 2016/45)**

(54) **HYGIENE ARTICLES AND PROCESSES FOR MAKING THE SAME**

HYGIENEARTIKEL UND VERFAHREN ZUR HERSTELLUNG DAVON

ARTICLES D'HYGIÈNE ET PROCÉDÉS DE FABRICATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **07.05.2015   US 201562158235 P**

(43) Date of publication of application:
**28.02.2018   Bulletin 2018/09**

(73) Proprietor: **ExxonMobil Chemical Patents Inc.**
**Baytown, TX 77520 (US)**

(72) Inventors:
• **TANG, Qi**
**Shangai 200023 (CN)**
• **VAN LOON, Achiel, J. M.**
**2900 Schoten - Antwerp (BE)**

(74) Representative: **ExxonMobil Chemical Europe Inc.**
**IP Law Europe**
**Hermeslaan 2**
**1831 Machelen (BE)**

(56) References cited:
**EP-A2- 0 307 116          WO-A1-02/090426**
**WO-A1-2012/058789     WO-A1-2015/085446**
**WO-A1-2015/200445     US-A1- 2012 100 356**

**Description**

[Deleted]

## FIELD OF THE INVENTION

The invention relates to hygiene articles, such as diapers and sanitary napkins, with improved backsheets. In particular, the backsheets comprise polyethylene polymers having certain properties.

## BACKGROUND

Hygiene products such as disposable diapers for infants and adults, incontinent pads, sanitary napkins, and pantiliners constitute major industries and serve important functions for different demographics of the population. In general, such hygiene products are made from a skin-facing layer or an inner topsheet (also called a cover or front sheet) which is liquid-permeable to facilitate entry of the fluid exudate from the wearer into the hygiene product, a core of highly absorbent material for absorbing liquid received through the topsheet, and an outer backsheet formed of a liquid impermeable plastic to eliminate leakage of fluid from the hygiene product. The backsheet may be vapor impermeable or vapor permeable. If vapor permeable, the product is said to be "breathable".

The typical trend in backsheets in recent years is downgauging to save material cost, usually by making the backsheets out of thinner films. However, the stiffness of the backsheet, which is important to maintain machinability of the film, drops while the film becomes thinner. Machinability is important for printing, lamination, and the assembly process of hygiene products. This aspect of the products allows manufacturers to differentiate their products in premium markets and affects overall production rates. The common approach to compensate for this stiffness loss is to use a higher density polymer but the consequence is that the film mechanical strength also drops or the end users feel that the film is too stiff or noisy to use, especially when softness is an important feature for hygiene products. Background references include U.S. Patent Nos. 4,341,216; 5,171,239; 5,928,209; 7,501,357; US 2013/0295364; US 2010/0062231; WO 01/51546, and WO 2014/123797.

WO2012/058789 discloses multilayer polymeric films comprising at least one layer A comprising polypropylene and at least one layer B comprising polyethylene, and at least one of layers A and B further comprising at least one copolymer of propylene and 5 to 30 weight % ethylene and/or C4 to C10 alpha-olefin.

Thus, there remains a need to make hygiene articles with improved backsheets that demonstrate a desired stiffness, especially for printed or embossed hygiene products, that also provide for a good balance of tensile properties and toughness, and processability.

## SUMMARY

In a class of embodiments, the invention provides for a hygiene article comprising:

    a) at least one top sheet;
    b) at least one absorbent core; and
    c) at least one backsheet;

wherein the at least one backsheet comprises from 3 wt% to 60 wt% of a first polyethylene polymer, based upon the total weight of the at least one backsheet, and wherein the first polyethylene polymer has a melt index (MI) from 0.2 dg/min to 3.0 dg/min; a density from 0.918 g/cm$^3$ to 0.945 g/cm$^3$; and a melt index ratio (MIR) from 25 to 60, wherein the at least one backsheet comprises a second polyethylene polymer.

In another class of embodiments, the invention provides for a process to produce a hygiene article, the process comprising disposing the following layers, in order, unto one another to produce the hygiene article:

    a) at least one top sheet;
    b) at least one absorbent core; and
    c) at least one backsheet;

wherein the at least one backsheet comprises from 3 wt% to 60 wt% of a first polyethylene polymer, based upon the total weight of the at least one backsheet, and wherein the first polyethylene polymer has a melt index (MI) from 0.2 dg/min to 3.0 dg/min; a density from 0.918 g/cm$^3$ to 0.945 g/cm$^3$; and a melt index ratio (MIR) from 25 to 60, wherein the at least one backsheet comprises a second polyethylene polymer.

Other embodiments of the invention are described and claimed herein and are apparent by the following disclosure.

## DETAILED DESCRIPTION

Before the present articles, compounds, components, compositions, devices, softwares, hardwares, equipments, configurations, schematics, systems, and/or methods or processes are disclosed and described, it is to be understood that unless otherwise indicated this invention is not limited to specific articles, compounds, components, compositions, devices, softwares, hardwares, equipments, configurations, schematics, systems, methods, processes, or the like, as such may vary, unless otherwise specified. It is also to be understood that the terminology used herein is for the purpose of describing particular embodiments only and is not intended to be limiting.

It must also be noted that, as used in the specification and the appended claims, the singular forms "a," "an" and "the" include plural referents unless otherwise specified.

Hygiene articles generally comprise a) at least one top sheet; b) at least one absorbent core; and c) at least one backsheet. The backsheet generally has a body-facing side oriented towards the body of the wearer of the article and a garment-facing side oriented towards the undergarment of the wearer of the article. The at least one backsheet comprises from 3 wt% to 60 wt% of a first polyethylene polymer, based upon the total weight of the at least one backsheet, and the first polyethylene polymer has a melt index (MI) from 0.2 dg/min to 3.0 dg/min; a density from 0.920 g/cm$^3$ to 0.945 g/cm$^3$; and a melt index ratio (MIR) from 25 to 60.

The hygiene articles are useful items and may be diapers (e.g., infants and adults), sanitary pads, sanitary napkins, and pantiliners. An exemplary multi-layered hygiene article is provided below, having seven total layers. The number of layers is not critical and may vary from several layers, for example, three or more, to many more layers, for example, five or more, ten or more, fifteen or more, and twenty or more layers.

| 1 | Hydrophilic and soft non-woven layer (sometimes referred to as the top sheet for certain applications) |
|---|---|
| 2/3/4/5 | Acquisition layer, Absorbent Material (often wrapped) |
| 6 | Back Sheet |
| 7 | Non-woven material |

### Topsheet

The topsheet is typically the layer of the hygiene article which is oriented towards and contacts the body of the wearer, and is therefore the first layer to receive the bodily discharges. The topsheet is normally made of a single layer, but may also comprise more than one layer (e.g., a central topsheet layer and two overlapping lateral stripes). For example, a single non-woven material may be used but the topsheet may be composed of several layers and treated to become hydrophilic so the liquid can pass through.

The topsheet is normally permeable to liquids, i.e., allow liquids to pass through the topsheet without significantly retarding or obstructing the transmission of such liquids therethrough.

Any conventional topsheet materials may be used. Suitable topsheets may be made, for example, from non-woven materials or perforated polyolefinic films. An exemplary topsheet is a relatively hydrophobic 20 gsm spunbonded nonwoven web comprising bicomponent fibers of the sheath/core type (e.g., polypropylene/polyethylene polymers). If desired, the topsheet may be treated with a surfactant to enhance liquid penetration to the core. The surfactant may be non-ionic and should be nonirritating to the skin. The topsheet may have a plurality of apertures to permit liquids deposited thereon to pass through to the core more quickly.

### Absorbent Core

The hygiene articles further comprise an absorbent core disposed between the topsheet and the backsheet. As used herein, the term "absorbent core" refers to a material or combination of materials suitable for absorbing, trapping, distributing, and/or storing fluids, for example, urine, blood, menses, and/or other exudates. It may optionally be separately wrapped.

The size and shape of the absorbent core may be such that the surface of the core in the horizontal plane is substantially smaller than the surface of the topsheet. By "substantially smaller", it is meant that the surface of the absorbent core is at least about 10% smaller than the surface of the topsheet or at least about 25% smaller than the surface of the topsheet. The absorbent core may be generally centered in the middle of the article. The absorbent core may be disposed away from the periphery of the article to provide improved flexibility along the edges of the article.

By providing an absorbent core having a substantially smaller surface than the topsheet, several benefits may be achieved. The amount of core material used is reduced, lowering the overall costs of manufacturing the article. A core having a smaller surface also increases the overall flexibility of the article because the regions of the article not provided with a core are generally less rigid than the region where the core is situated.

The absorbent core may be fashioned into many shapes, for example, rounded, oval, rectangular, and square. For example, it is typical for absorbent cores to be rectangular for ease of manufacturing. However, flexibility and compatibility with various styles of undergarments may be better with cores having a curved shape (such as an oval shape) without comprising right angles.

The absorbent core may be made of any suitable materials. Nonlimiting examples of suitable liquid-absorbent materials include comminuted wood pulp which is generally referred to as airfelt; creped cellulose wadding; absorbent gelling materials including superabsorbent polymers such as hydrogel-forming polymeric gelling agents; chemically stiffened, modified, or cross-linked cellulose fibers; meltblown polymers including co- form; synthetic fibers including crimped polyester fibers; tissue including tissue wraps and tissue laminates; capillary channel fibers; absorbent foams; absorbent sponges; synthetic staple fibers; peat moss; foamed polyethylene or polypropylene compositions; nonwoven polyethylene or polypropylene materials; or any equivalent material; or combinations thereof. The absorbent core can comprise superabsorbent polymers (SAP), normally distributed within a matrix of cellulosic fibers, for example, in order to reduce the thickness of the absorbent core.

The absorbent core can be a monolayer or can be a laminate of two or more layers. For example, the core may comprise a fluid impermeable barrier layer on its backsheet-facing side to prevent fluids retained by the absorbent core from striking through the hygiene article. General information regarding absorbent cores may be found in, for example, WO 2002/007662 and WO 1991/019471.

**Backsheet**

The general function of the backsheet is to prevent discharges absorbed by the core from escaping the hygiene article. The backsheet may include any suitable material. Typically, these materials are generally flexible, liquid resistant, and impermeable to liquids. The backsheet generally comprises at least a film that may be monolayer or two or more layers. The films may be cast or blown films, optionally, embossed and/or oriented or stretched, in either direction: Machine Direction (MD) or Transverse Direction (TD), either on-line or off-line. The films may also be co-extruded. This backsheet may also contain fillers, pigment, various additives, and any combination thereof. The backsheet may also be a laminate with non-woven fabric.

In a class of embodiments, the at least one backsheet may comprise from 3 wt% to 60 wt% of a first polyethylene polymer (as described below), based upon the total weight of the at least one backsheet, and the first polyethylene polymer may have a melt index (MI) from 0.2 dg/min to 3.0 dg/min; a density from .920 g/cm$^3$ to .945 g/cm$^3$; and a melt index ratio (MIR) from 25 to 60.

Alternatively, the at least one backsheet may comprise from 5 wt% to 75 wt%, alternatively, from 10 wt% to 40 wt%, alternatively, from 15 wt% to 25 wt%, alternatively, from 20 wt% to 30 wt%, and alternatively, about 22 wt% of the first polyethylene polymer, based upon the total weight of the backsheet.

In another class of embodiments, the at least one backsheet may comprise from 75 wt% to 5 wt%, alternatively, from 40 wt% to 10 wt%, alternatively, from 30 wt% to 15 wt%, and alternatively, about 22 wt% of a second polyethylene polymer (as described below), based upon the total weigh of the backsheet.

In other embodiments, the at least one backsheet may comprise from 15 wt% to 25 wt% of the first polyethylene polymer and from 25 wt% to 15 wt% of the second polyethylene polymer, optionally, with a third polyethylene polymer (as described below) and/or a propylene polymer (as described below), based upon the total weight of the at least one backsheet.

In any of the embodiments described above, the at least one backsheet may comprise up to 75 wt% of a filler, such as, for example, calcium carbonate, based upon the total weight of the at least one backsheet. Alternatively, the at least one backsheet may comprise up to 60 wt% of a filler, alternatively, up to 50 wt% of a filler, alternatively, up to 40 wt% of a filler, alternatively, up to 30 wt% of a filler, based upon the total weight of the at least one backsheet.

In other embodiments, the at least one backsheet may comprise from 10 wt% to 75 wt% of a filler, alternatively, from 20 wt% to 60 wt% of a filler, alternatively, from 30 wt% to 55 wt% of a filler, alternatively, from 40 wt% to 55 wt% of a filler, and alternatively, from 45 wt% to 55 wt% of a filler, based upon the total weight of the at least one backsheet.

Typically, a thinner backsheet film is desired to save on material costs and for design considerations of the final article. For example, the backsheet film may have an average backsheet thickness of 24 μm or less, alternatively, 20 μm or less, 15 μm or less, alternatively, 14 μm or less, 12 μm or less, 11 μm or less, and alternatively, 10 μm or less. As used herein "average backsheet thickness" refers to the thickness of a film, typically, expressed in microns of the film, prior to any additional conversion or treatments such as embossing. As thickness measurements on embossed films are difficult to obtain, the gauge of the film is may be expressed in gram per square meter or gsm, equal to the weight of one square meter of film. Typically, 10 pieces of 10 square centimeters each are cut out of the film and the weight is multiplied by 10 to obtain gsm.

Thus, alternatively, the backsheet film may have an average backsheet thickness of 35 grams per square meter (gsm) or less, alternatively, 30 gsm or less, 25 gsm or less, 20 gsm or less, 15 gsm or less, 12 gsm or less, or 10 gsm or less.

Although embodiments of this invention are directed to both "breathable" and regular hygiene articles, those of the breathable nature tend to extract premiums in the market place. Thus, backsheets which are permeable to vapor are known as breathable backsheets and are used in breathable hygiene articles. These breathable backsheets provide a cooler garment and permit some drying of the article while being worn. In general, these breathable backsheets are intended to allow the passage of vapor, typically expressed as Water Vapor Transmission Rate or WVTR through them while retarding the passage of liquid.

Breathable backsheets may be obtained by creating microvoids in one or more layers of the backsheet. Microvoids may be created by the incorporation of fillers, such as calcium carbonate ($CaCO_3$) or other suitable materials, into one or more layers of the backsheet followed by an orientation or stretching process. Other suitable materials include organic fillers such as polystyrene in polyethylene or water-swellable fillers such as silica or hydrogel.

The backsheet has a garment-facing side and an opposite body-facing side. The garment-facing side of the backsheet can comprise a non-adhesive area and an adhesive area. The adhesive area may be provided by any conventional means. Pressure sensitive adhesives have been commonly found to work well for this purpose.

In several classes of embodiments, the at least one backsheet has a 5% yield strength MD or yield force MD of 15 MPa or greater and/or a 10% yield strength MD or yield force MD of 20 MPa or greater in accordance with ASTM D 822. Alternatively, the at least one backsheet has a 5% yield strength MD of 17 MPa or greater, 18 MPa or greater, or 20 MPa or greater, and a 10% yield strength MD of 22 MPa or greater, 24 MPa or greater, or 25 MPa or greater.

In other embodiments, the at least one backsheet has a Yield Strength MD of 11 MPa or greater, 12 MPa or greater, 13 MPa or greater, or 15 MPa or greater, in accordance with ASTM D 822.

**Polymers**

An "olefin", alternatively referred to as "alkene", is a linear, branched, or cyclic compound of carbon and hydrogen having at least one double bond. For purposes of this specification and the claims appended thereto, when a polymer or copolymer is referred to as comprising an olefin, including, but not limited to ethylene, propylene, hexene, octane, diene, and mixtures thereof, the olefin present in such polymer or copolymer is the polymerized form of the olefin. For example, when a copolymer is said to have an "ethylene" content of 35 wt% to 55 wt%, it is understood that the mer unit in the copolymer is derived from ethylene in the polymerization reaction and said derived units are present at 35 wt% to 55 wt%, based upon the weight of the copolymer.

A "polymer" has two or more of the same or different mer units. A "homopolymer" is a polymer having monomer units (or "mer" units as a shorthand) that are the same. A "copolymer" is a polymer having two or more mer units that are different from each other. A "terpolymer" is a polymer having three mer units that are different from each other. An "interpolymer" is a polymer having multiple different mer units that are different from each other. Block copolymers are also contemplated. The term "different" as used to refer to mer units indicates that the mer units differ from each other by at least one atom or are different isomerically. Accordingly, the definition of copolymer, as used herein, includes terpolymers and the like. Likewise, the definition of polymer, as used herein, includes copolymers and the like. Thus, as used herein, the terms "polyethylene", "ethylene polymer", "ethylene copolymer", and "ethylene-based polymer" mean a polymer or copolymer comprising at least 50 mol% ethylene units (preferably at least 70 mol% ethylene units, more preferably at least 80 mol% ethylene units, even more preferably at least 90 mol% ethylene units, even more preferably at least 95 mol% ethylene units or 100 mol% ethylene units (in the case of a homopolymer)). Furthermore, the term "polyethylene composition" includes a blend containing one or more polyethylene components.

**First Polyethylene Polymer**

The first polyethylene polymers are ethylene-based polymers having about 99.0 to about 80.0 wt%, 99.0 to 85.0 wt%, 99.0 to 87.5 wt%, 99.0 to 90.0 wt%, 99.0 to 92.5 wt%, 99.0 to 95.0 wt%, or 99.0 to 97.0 wt%, of polymer units derived from ethylene and about 1.0 to about 20.0 wt%, 1.0 to 15.0 wt%, 1.0 to 12.5 wt%, 1.0 to 10.0 wt%, 1.0 to 7.5 wt%, 1.0 to 5.0 wt%, or 1.0 to 3.0 wt% of polymer units derived from one or more $C_3$ to $C_{20}$ α-olefin comonomers, preferably $C_3$ to $C_{10}$ α-olefins, and more preferably $C_4$ to $C_8$ α-olefins, such as hexene and octene. The α-olefin comonomer may be linear or branched, and two or more comonomers may be used, if desired. Examples of suitable comonomers include propylene, butene, 1-pentene; 1-pentene with one or more methyl, ethyl, or propyl substituents; 1-hexene; 1-hexene with one or more methyl, ethyl, or propyl substituents; 1-heptene; 1-heptene with one or more methyl, ethyl, or propyl substituents; 1-octene; 1-octene with one or more methyl, ethyl, or propyl substituents; 1-nonene; 1-nonene with one or more methyl, ethyl, or propyl substituents; ethyl, methyl, or dimethyl-substituted 1-decene; 1-dodecene; and styrene. Particularly suitable comonomers include 1-butene, 1-hexene, and 1-octene, 1-hexene being most preferred.

Typically, the first polyethylene polymer may have a Composition Distribution Breadth Index (CDBI) of at least 70%, e.g., $\geq$ about 75%, $\geq$ about 80%, $\geq$ about 82, $\geq$ about 85, $\geq$ about 87, $\geq$ about 90.0%, $\geq$ about 95%, or $\geq$ about 98%. Additionally or alternatively, the CDBI may be $\leq$ 100%, e.g., $\leq$ about 98%, $\leq$ about 95%, $\leq$ about 90%, $\leq$ about 87%, $\leq$ about 85%, $\leq$ about 82%, $\leq$ about 80%, or $\leq$ about 75%. Ranges expressly disclosed include, but are not limited to, ranges formed by combinations any of the above-enumerated values, e.g., 70 to about 98%, about 80 to about 95%, about 85 to about 90% etc.

The first polyethylene polymer may have a melt index, $I_{2.16}$, according to ASTM D1238 (190°C/2.16 kg), of $\geq$ about 0.10 g/10 min., e.g., $\geq$ about 0.15 g/10 min., $\geq$ about 0.18 g/10 min., $\geq$ about 0.20 g/10 min., $\geq$ about 0.22 g/10 min., $\geq$ about 0.25 g/10 min., $\geq$ about 0.28 g/10 min, or $\geq$ about 0.30 g/10 min and, also, a melt index ($I_{2.16}$) $\leq$ about 3.00 g/10 min., e.g., $\leq$ about 2.00 g/10 min., $\leq$ about 1.00 g/10 min., $\leq$ about 0.70 g/10 min., $\leq$ about 0.50 g/10 min., $\leq$ about 0.40 g/10 min., or $\leq$ about 0.30 g/10 min. Ranges expressly disclosed include, but are not limited to, ranges formed by combinations any of the above-enumerated values, e.g., about 0.10 to about 0.30, about 0.15 to about 0.25, about 0.18 to about 0.22 g/10 min., etc.

The first polyethylene polymer may have a melt index ratio (MIR) from 25 to 60, alternatively, from 30 to 55, alternatively, from 35 to 50, and alternatively, from 40 to 46. MIR is defined as $I_{21.6}/I_{2.16}$ according to ASTM D1238 at 190°C.

The first polyethylene polymer may have a melt strength of from 0.01 cN to 1.0 cN, alternatively, from 0.02 cN to 0.1 cN. Melt Strength (MS) is measured at 190°C with a Goettfert Rheotens melt strength apparatus in conjunction with an Instron capillary Rheometer. In particular, a polymer melt strand is extruded from a capillary die and gripped between two counter rotating wheels on the apparatus whose speed is increased at a constant acceleration and controlled by the Acceleration Programmer (Model 45917, at a setting of 12). The maximum pulling force (in units of cN) achieved before the strand brakes or starts to show draw resonance is the melt strength. The capillary die has a length of one inch (2.54 cm) and a diameter of 0.06 inches (0.15 cm). The polymer melt is extruded from the die at a speed of 3 inches/min (7.62 cm/min). The distance between the die exit and the wheel contact point should be 3.94 inches (100 mm).

The first polyethylene polymer may have a density about 0.918 g/cm$^3$ $\geq$ about 0.920 g/cm$^3$, e.g., $\geq$ about 0.922 g/cm$^3$, $\geq$ about 0.928 g/cm$^3$, $\geq$ about 0.930 g/cm$^3$, $\geq$ about 0.932 g/cm$^3$. Additionally, the first polyethylene polymer may have a density $\leq$ about 0.945 g/cm$^3$, e.g., $\leq$ about 0.940 g/cm$^3$, $\leq$ about 0.937 g/cm$^3$, $\leq$ about 0.935 g/cm$^3$, $\leq$ about 0.933 g/cm$^3$, or $\leq$ about 0.930 g/cm$^3$. Ranges expressly disclosed include, but are not limited to, ranges formed by combinations any of the above-enumerated values, e.g., about 0.920 to about 0.945 g/cm$^3$, 0.920 to 0.930 g/cm$^3$, 0.925 to 0.935 g/cm$^3$, 0.920 to 0.940 g/cm$^3$, etc. Density is determined using chips cut from plaques compression molded in accordance with ASTM D-1928 Procedure C, aged in accordance with ASTM D-618 Procedure A, and measured as specified by ASTM D-1505.

Typically, the first polyethylene polymer may have a molecular weight distribution (MWD, defined as $M_w/M_n$) of about 2.5 to about 5.5, preferably 4.0 to 5.0 and about 4.4 to 5.0.

The first polyethylene polymer may also be characterized by an averaged 1% secant modulus (M) of from 10,000 to 60,000 psi (pounds per square inch), and a relation between M and the dart drop impact strength in g/mil (1 mil = 25.4 micron) (DIS) complying with formula (A):

$$DIS \geq 0.8 * [100 + e^{(11.71 - 0.000268M + 2.183 \times 10^{-9} M^2)}], \qquad (A)$$

where "e" represents 2.7183, the base Napierian logarithm, M is the averaged modulus in psi, and DIS is the 26 inch dart impact strength. The DIS is preferably from about 120 to about 1000 g/mil, even more preferably, from about 150 to about 800 g/mil.

The relationship of the Dart Impact Strength to the averaged 1% secant modulus is thought to be one indicator of long-chain branching in the ethylene-based polymer. Thus, alternatively ethylene-based polymers of certain embodiments may be characterized as having long-chain branches. Long-chain branches for the purposes of this invention represent the branches formed by reincorporation of vinyl-terminated macromers, not the branches formed by incorporation of the comonomers. The number of carbon atoms on the long-chain branches ranges from a chain length of at least one carbon more than two carbons less than the total number of carbons in the comonomer to several thousands. For example, a long-chain branch of an ethylene/hexene ethylene-based polymer is at least five (5) carbons in length (i.e., 6 carbons less 2 equals 4 carbons plus one equals a minimum branch length of five carbons for long-chain branches).

Particular ethylene-based polymers have a 0.05 to 1.0, particularly 0.05 to 0.5, 0.1 to 0.4, or 0.2 to 0.3, long-chain branches per 1000 carbon atoms. Ethylene-based polymers having levels of long-chain branching greater than 1.0 long-chain branch per 1000 carbon atoms may have some beneficial properties, e.g., improved processability, shear thinning, and/or delayed melt fracture, and/or improved melt strength.

Various methods are known for determining the presence of long-chain branches. For example, long-chain branching can be determined using $^{13}C$ nuclear magnetic resonance (NMR) spectroscopy and to a limited extent; e.g., for ethylene homopolymers and for certain copolymers, and it can be quantified using the method of Randall (Journal of Macromolecular Science, Rev. Macromol. Chem. Phys., C29 (2&3), p. 285-297). Although conventional $^{13}C$ NMR spectroscopy cannot determine the length of a long-chain branch in excess of about six carbon atoms, there are other known techniques useful for quantifying or determining the presence of long-chain branches in ethylene-based polymers, such as ethylene/1-octene interpolymers. For those ethylene-based polymers wherein the $^{13}C$ resonances of the comonomer overlap completely with the $^{13}C$ resonances of the long-chain branches, either the comonomer or the other monomers (such as ethylene) can be isotopically labeled so that the long-chain branches can be distinguished from the comonomer. For example, a copolymer of ethylene and 1-octene can be prepared using $^{13}C$-labeled ethylene. In this case, the resonances associated with macromer incorporation will be significantly enhanced in intensity and will show coupling to neighboring $^{13}C$ carbons, whereas the octene resonances will be unenhanced.

Alternatively, the degree of long-chain branching in ethylene-based polymers may be quantified by determination of the branching index. The branching index g' is defined by the following equation:

$$g' = \left. \frac{IV_{Br}}{IV_{Lin}} \right|_{M_w}$$

where g' is the branching index, $IV_{Br}$ is the intrinsic viscosity of the branched ethylene-based polymer and $IV_{Lin}$ is the intrinsic viscosity of the corresponding linear ethylene-based polymer having the same weight average molecular weight and molecular weight distribution as the branched ethylene-based polymer, and in the case of copolymers and terpolymers, substantially the same relative molecular proportion or proportions of monomer units. For the purposes, the molecular weight and molecular weight distribution are considered "the same" if the respective values for the branched polymer and the corresponding linear polymer are within 10% of each other. Preferably, the molecular weights are the same and the MWD of the polymers are within 10% of each other. A method for determining intrinsic viscosity of polyethylene is described in Macromolecules, 2000, 33, 7489-7499. Intrinsic viscosity may be determined by dissolving the linear and branched polymers in an appropriate solvent, e.g., trichlorobenzene, typically measured at 135°C. Another method for measuring the intrinsic viscosity of a polymer is ASTM D-5225-98 - Standard Test Method for Measuring Solution Viscosity of Polymers with a Differential Viscometer, which is incorporated by reference herein in its entirety.

The branching index, g' is inversely proportional to the amount of branching. Thus, lower values for g' indicate relatively higher amounts of branching. The amounts of short and long-chain branching each contribute to the branching index according to the formula: $g'=g'_{LCB} \times g'_{SCB}$. Thus, the branching index due to long-chain branching may be calculated from the experimentally determined value for g' as described by Scholte, et al., in J. App. Polymer Sci., 29, pp. 3763-3782 (1984).

Typically, the first polyethylene polymer may have a $g'_{vis}$ of 0.85 to 0.98, particularly, 0.87 to 0.97, 0.89 to 0.97, 0.91 to 0.97, 0.93 to 0.95, 0.97 to 0.99, 0.97 to 0.98, or 0.95 to 0.98.

Suitable commercial polymers for the first polyethylene polymer are available from ExxonMobil Chemical Company as Enable™ metallocene polyethylene (mPE) resins.

**Second Polyethylene Polymer**

The hygiene article may comprise a second polyethylene polymer in one or more of its top sheet, absorbent core, or backsheet. The second polyethylene polymers are ethylene-based polymers comprising ≥ 50.0 wt% of polymer units derived from ethylene and ≤ 50.0 wt% preferably 1.0 wt% to 35.0 wt%, even more preferably 1 to 6 wt% of polymer units derived from a $C_3$ to $C_{20}$ alpha-olefin comonomer (for example, hexene or octene).

The second polyethylene polymer may have a composition distribution breadth index (CDBI) ≥ 60.0%, e.g., ≥ about 65%, ≥ about 70%, ≥ about 72, ≥ about 75, ≥ about 77, ≥ about 80.0%, or ≥ about 90%. Alternatively, the CDBI may be ≤ 100%, e.g., ≤ about 90%, ≤ about 85%, ≤ about 80%, ≤ about 77%, ≤ about 75%, ≤ about 72%, ≤ about 70%, or ≤ about 65%. Ranges expressly disclosed include, but are not limited to, ranges formed by any combinations of the above-enumerated values, e.g., 60 to 100%, 60.0 to about 80.0%, or about 65 to about 80%.

The second polyethylene polymer may have a density of ≥ about 0.910 g/cm$^3$, ≥ about 0.915 g/cm$^3$, ≥ about

$0.920 \text{ g/cm}^3$, $\geq$ about $0.925 \text{ g/cm}^3$, $\geq$ about $0.930 \text{ g/cm}^3$, or $\geq$ about $0.940 \text{ g/cm}^3$. Alternatively, the second polyethylene polymer may have a density of $\leq$ about $0.950 \text{ g/cm}^3$, e.g., $\leq$ about $0.940 \text{ g/cm}^3$, $\leq$ about $0.930 \text{ g/cm}^3$, $\leq$ about $0.925$ $\text{g/cm}^3$, $\leq$ about $0.920 \text{ g/cm}^3$, or $\leq$ about $0.915 \text{ g/cm}^3$. Ranges expressly disclosed include ranges formed by combinations any of the above-enumerated values, e.g., 0.910 to $0.950 \text{ g/cm}^3$, 0.910 to $0.930 \text{ g/cm}^3$, 0.910 to $0.925 \text{ g/cm}^3$, etc. Density is determined using chips cut from plaques compression molded in accordance with ASTM D-1928 Procedure C, aged in accordance with ASTM D-618 Procedure A, and measured as specified by ASTM D-1505.

The second polyethylene polymer may have a melt index ($I_{2.16}$) according to ASTM D1238 (190°C/2.16 kg) of $\geq$ about 0.5 g/10 min., e.g., $\geq$ about 0.5 g/10 min., $\geq$ about 0.7 g/10 min., $\geq$ about 0.9 g/10 min., $\geq$ about 1.1 g/10 min., $\geq$ about 1.3 g/10 min., $\geq$ about 1.5 g/10 min., or $\geq$ about 1.8 g/10 min. Alternatively, the melt index ($I_{2.16}$) may be $\leq$ about 8.0 g/10 min., $\leq$ about 7.5 g/10 min., $\leq$ about 5.0 g/10 min., $\leq$ about 4.5 g/10 min., $\leq$ about 3.5 g/10 min., $\leq$ about 3.0 g/10 min., $\leq$ about 2.0 g/10 min., e.g., $\leq$ about 1.8 g/10 min., $\leq$ about 1.5 g/10 min., $\leq$ about 1.3 g/10 min., $\leq$ about 1.1 g/10 min., $\leq$ about 0.9 g/10 min., or $\leq$ about 0.7 g/10 min., 0.5 to 2.0 g/10 min., particularly 0.75 to 1.5 g/10 min. Ranges expressly disclosed include ranges formed by combinations any of the above-enumerated values, e.g., about 0.5 to about 8.0 g/10 min., about 0.7 to about 1.8 g/10 min., about 0.9 to about 1.5 g/10 min., about 0.9 to 1.3, about 0.9 to 1.1 g/10 min, about 1.0 g/10 min., etc.

In particular embodiments, the second polyethylene polymer may have a density of 0.910 to $0.920 \text{ g/cm}^3$, a melt index ($I_{2.16}$) of 0.5 to 8.0 g/10 min., and a CDBI of 60.0% to 80.0%, preferably between 65% and 80%.

The second polyethylene polymers are generally considered linear, meaning they have a $g'_{vis} \geq 0.98$, as discussed herein. Suitable second such polyethylene polymers are available from ExxonMobil Chemical Company under the trade name Exceed™ metallocene (mPE) resins. The MIR for EXCEED materials will typically be from about 15 to about 20.

**Third Polyethylene Polymer**

The hygiene article may comprise a third polyethylene polymer in one or more of its top sheet, absorbent core, or backsheet. The third polyethylene polymer may be an ethylene-based polymer having a $g'_{vis}$ as described above of 0.50 to 0.85, particularly 0.50 to 0.80, 0.50 to 0.75, 0.50 to 0.70, 0.50 to 0.65, 0.50 to 0.60, or 0.50 to 0.55.

Suitable third polyethylene polymer may be a copolymer of ethylene, and one or more polar comonomers or $C_3$ to $C_{10}$ $\alpha$-olefins. Typically, the third polyethylene polymer useful herein includes 99.0 to about 80.0 wt%, 99.0 to 85.0 wt%, 99.0 to 87.5 wt%, 95.0 to 90.0 wt%, of polymer units derived from ethylene and about 1.0 to about 20.0 wt%, 1.0 to 15.0 wt%, 1.0 to 12.5 wt%, or 5.0 to 10.0 wt% of polymer units derived from one or more polar comonomers, based upon the total weight of the polymer. Suitable polar comonomers include, but are not limited to: vinyl ethers such as vinyl methyl ether, vinyl n-butyl ether, vinyl phenyl ether, vinyl beta-hydroxy-ethyl ether, and vinyl dimethylamino-ethyl ether; olefins such as propylene, butene-1, cis-butene-2, trans-butene-2, isobutylene, 3,3,-dimethylbutene-1, 4-methylpentene-1, octene-1, and styrene; vinyl type esters such as vinyl acetate, vinyl butyrate, vinyl pivalate, and vinylene carbonate; haloolefins such as vinyl fluoride, vinylidene fluoride, tetrafluoroethylene, vinyl chloride, vinylidene chloride, tetrachloroethylene, and chlorotrifluoroethylene; acrylic-type esters such as methyl acrylate, ethyl acrylate, n-butyl acrylate, t-butyl acrylate, 2-ethylhexyl acrylate, alpha-cyanoisopropyl acrylate, beta-cyanoethyl acrylate, o-(3-phenylpropan-1,3,-dionyl)phenyl acrylate, methyl methacrylate, n-butyl methacrylate, t-butyl methacrylate, cyclohexyl methacrylate, 2-ethylhexyl methacrylate, methyl methacrylate, glycidyl methacrylate, beta-hydroxethyl methacrylate, beta-hydroxpropyl methacrylate, 3-hydroxy-4-carbo-methoxy-phenyl methacrylate, N,N-dimethylaminoethyl methacrylate, t-butylaminoethyl methacrylate, 2-(1-aziridinyl)ethyl methacrylate, diethyl fumarate, diethyl maleate, and methyl crotonate; other acrylic-type derivatives such as acrylic acid, methacrylic acid, crotonic acid, maleic acid, methyl hydroxy maleate, itaconic acid, acrylonitrile, fumaronitrile, N,N-dimethylacrylamide, N-isopropylacrylamide, N-t-butylacrylamide, N-phenylacrylamide, diacetone acrylamide, methacrylamide, N-phenylmethacrylamide, N-ethylmaleimide, and maleic anhydride; and other compounds such as allyl alcohol, vinyltrimethylsilane, vinyltriethoxysilane, N-vinylcarbazole, N-vinyl-N-methylacetamide, vinyldibutylphosphine oxide, vinyldiphenylphosphine oxide, bis-(2-chloroethyl) vinylphosphonate, and vinyl methyl sulfide.

In some embodiments, the third polyethylene polymer is an ethylene/vinyl acetate copolymer having about 2.0 wt% to about 15.0 wt%, typically about 5.0 wt% to about 10.0 wt%, polymer units derived from vinyl acetate, based on the amounts of polymer units derived from ethylene and vinyl acetate (EVA). In certain embodiments, the EVA resin can further include polymer units derived from one or more comonomer units selected from propylene, butene, 1-hexene, 1-octene, and/or one or more dienes.

Suitable dienes include, for example, 1,4-hexadiene, 1,6-octadiene, 5-methyl-1,4-hexadiene, 3,7-dimethyl-1,6-octadiene, dicyclopentadiene (DCPD), ethylidene norbornene (ENB), norbornadiene, 5-vinyl-2-norbornene (VNB), and combinations thereof.

The third polyethylene polymer may have a melt index ($I_{2.16}$) as measured according to ASTM D1238, 2.16 kg, 190°C, of 0.1 to 12.0 g/10 min., particularly 0.1 to 2.5 g/10 min., 0.2 to 1.0, or 0.3 to 0.7 g/10 min.

In some embodiments, the third polyethylene polymer may have a melting point of 40°C or less, as measured by industry acceptable thermal methods, such as Differential Scanning Calorimetry (DSC). In other embodiments, the melting point may be 40.0°C to about 90.0°C.; 40.0°C to 80.0°C; 50.0°C to 70.0°C; 55.0°C to 65.0°C; or about 60.0°C.

The third polyethylene polymer may have a Vicat softening point of about 20.0°C to about 80.0°C, as measured by ASTM D1525. The Vicat softening point can also range from a low of about 20°C, 25.0°C, or 30.0°C to a high of about 35.0°C, 40.0°C, or 50.0°C. The Vicat softening point of the High Pressure Poly Ethylene (HPPE) resin can also be 20.0°C to 70.0°C; 30.0°C to 60.0°C; 35.0°C to 45.0°C; about 35.0°C, or 40.0°C and is also contemplated.

The third polyethylene polymers are available from ExxonMobil Chemical Company as ExxonMobil™ LDPE or Nexxstar™ resins.

A fourth polyethylene polymer may also be present as High Density Polyethylene or HDPE. The HDPE may be unimodal or bimodal/multimodal and have a narrow molecular weight distribution (MWD) or broad MWD.

## Propylene-Based Polymer

The hygiene article may comprise a propylene-base polymer or polypropylene polymer in one or more of its top sheet, absorbent core, or backsheet. The "polypropylene" that may be used in the article is preferably a homopolymer or copolymer comprising from 60 wt% or 70 wt% or 80 wt% or 85 wt% or 90 wt% or 95 wt% or 98 wt% or 99 wt% to 100 wt% propylene-derived units; comprising within the range of from 0 wt% or 1 wt% or 5 wt% to 10 wt% or 15 wt% or 20 wt% or 30 wt% or 40 wt% $C_2$ and/or $C_4$ to $C_{10}$ $\alpha$-olefin derived units. It may be made by any desirable process using any desirable catalyst as is known in the art, such as a Ziegler-Natta catalyst, a metallocene catalyst, or other single-site catalyst, using solution, slurry, high pressure, or gas phase processes.

In any case, useful polypropylene polymers have a DSC melting point (ASTM D 3418) of at least 130°C or 140°C or 150°C or 160°C or 165°C, or within a range of from 130°C or 135°C or 140°C to 150°C or 160°C or 170°C. A "highly crystalline" polypropylene is preferred in certain embodiments and is typically isotactic and comprises 100 wt% propylene-derived units (propylene homopolymer) and has a relatively high melting point of from greater than (greater than or equal to) 130°C or 140°C or 145°C or 150°C or 155°C or 160°C or 165°C.

The term "crystalline," as used herein, characterizes those polymers which possess high degrees of inter- and intra-molecular order. Preferably, the polypropylene polymer has a heat of fusion (Hf) greater than 60 J/g or 70 J/g or 80 J/g, as determined by DSC analysis. The heat of fusion is dependent on the type of the polypropylene polymer. The thermal energy for the highest order of polypropylene polymer is estimated at 189 J/g, that is, 100% crystallinity is equal to a heat of fusion of 189 J/g. A polypropylene homopolymer will have a higher heat of fusion than a copolymer or blend of homopolymer and copolymer. Also, the polypropylene polymers may have a glass transition temperature (ISO 11357-1, Tg), preferably, between -20°C or -10°C or 0°C to 10°C or 20°C or 40°C or 50°C. The polypropylene polymers may have a Vicat softening temperature (ISO 306, or ASTM D 1525) of greater than 120°C or 110°C or 105°C or 100°C, or within a range of from 100°C or 105°C to 110°C or 120°C or 140°C or 150°C, or a particular range of from 110°C or 120°C to 150°C.

The polypropylene polymer may have a melt flow rate ("MFR", 230°C, 2.16 kg, ASTM D1238) within the range from 10, or 18 g/10 min to 40, or 50, or 60, or 80, g/10 min. Also, the polypropylene polymer may have a molecular weight distribution (MWD) (determined by GPC) of from 1.5 or 2.0 or 2.5 to 3.0 or 3.5 or 4.0 or 5.0 or 6.0 or 8.0 in certain embodiments.

Suitable grades of polypropylene that are useful in the compositions described herein include those made by ExxonMobil, LyondellBasell, Total, Borealis, Japan Polypropylene, Mitsui, and other sources. A description of semi-crystalline polypropylene polymers may be found in "Polypropylene Handbook", (E. P. Moore Editor, Carl Hanser Verlag, 1996).

## Additives

The hygiene article may comprise one or more additives in one or more of its top sheet, absorbent core, or backsheet. Additives may be included in one or more components of the aforementioned as desired. Additives may be typically added from 3.0 wt% or less, alternatively, 2.5 wt% or less, alternatively, 2.0 wt% or less, and alternatively, 10000 ppm or less. In contrast, fillers and/or pigments may typically be added in levels from 0.5 wt% up to 60 wt%. Such additives are well known in the art, and can include, for example: antioxidants (e.g., hindered phenolics such as IRGANOX™ 1010 or IRGANOX™ 1076 available from Ciba-Geigy); anti-cling additives; slip agents (e.g., unsaturated aliphatic amides); tackifiers; such as polybutenes, terpene resins, aliphatic and aromatic hydrocarbon resins, alkali metal and glycerol stearates and hydrogenated rosins; UV stabilizers; heat stabilizers; antiblocking agents; release agents; anti-static agents; pigments; colorants; dyes; waxes; silica; fillers; talc; clay; dispersion agents (e.g., calcium stearate, zince stearate), titanium oxide; and the like.

## Methods of Manufacture

The hygiene articles of the present invention may be produced by any conventional means. The different layers may thus be assembled using standard means such as embossing (e.g. thermal bonding), ultrasonic bonding, gluing/using adhesives or any combination of the aforementioned. The converting line may comprise a printing step wherein the ink is applied to the backsheet of the article. The backsheet may also comprise dyes or colorants.

In a class of embodiments, the invention also provides for a process to produce a hygiene article, the process comprising disposing the following layers, in order, unto one another to produce the hygiene article:

a. at least one top sheet;

b. at least one absorbent core; and

c. at least one backsheet; wherein the at least one backsheet comprises from 3 wt% to 60 wt% of a first polyethylene polymer, based upon the total weight of the at least one backsheet, and wherein the first polyethylene polymer has a melt index (MI) from 0.2 dg/min to 3.0 dg/min; a density from 0.920 g/cm$^3$ to 0.945 g/cm$^3$; and a melt index ratio (MIR) from 25 to 60, wherein the at least one backsheet comprises a second polyethylene polymer.

The at least one backsheet may further be oriented, optionally, in the machine direction (MD) and/or transverse direction (TD). This may be done in-line with the film production or off-line on a stand alone unit.

In a class of embodiments, the at least one backsheet is cast film, optionally, a monolayer cast film or multilayer cast film.

In another class of embodiments, the at least one backsheet is blown film, *optionally,* a coextruded blown film. The blown film may be a monolayer blown film or a multilayer blown film.

In another class of embodiments, the at least one backsheet may be embossed, either in-line with film production or off line in a stand alone embossing unit. The embossing may be done with heated rollers or at ambient temperature.

## Examples

It is to be understood that while the invention has been described in conjunction with the specific embodiments thereof, the foregoing description is intended to illustrate and not limit the scope of the invention. Other aspects, advantages and modifications will be apparent to those skilled in the art to which the invention pertains.

Therefore, the following examples are put forth so as to provide those skilled in the art with a complete disclosure and description and are not intended to limit the scope of that which the inventors regard as their invention.

## Example 1

**Formulation #1 - 14gsm**

40% Exceed™ mPE 4518PA + 50% CaCO$_3$ + 8% ExxonMobil™ LDPE (7 MI) + 2% additives

**Formulation #2 - 14gsm**

20% Exceed™ mPE 4518PA + 20% Enable™ mPE 20 -10CB + 50% CaCO$_3$ + 8% ExxonMobil™ LDPE (7MI) + 2% additives

Formulations 1 and 2 were first compounded into a free flowing pelletized material on a standard extrusion compounding line at standard processing conditions. The additives referenced above like white pigment, athermal stabilizer, and processing aids.

After this extrusion step, the compounds were extruded on a typical mono-layer cast line, characterized by the following features.

Screw size : 80 mm, 30L/D

Die width : 1200 mm

Die gap : 1.4 mm

Line speed:76m/min

Stretch Ratio:2.4

After extrusion, the film was guided through an in-line heated MDO unit, stretching the initial film down to 14 gsm, followed by on-line embossing.

The following film properties were measured in Table 1.

## Table 1

| Formulaiton | Test Method | Unit | Formulation #1 | Formulation #2 | Market Reference |
|---|---|---|---|---|---|
| Thickness | | GSM | 14 | 14 | 15 |
| Mechanical properties: | | | | | |
| Tensile strenght | ASTM D-882 | | | | |
| - Yield Force at 10% strain MD | | mPa | 19.81 | 24.68 | 16.4 |
| - Yield Force at 10% strain TD | | mPa | 4.02 | 4.42 | N/A |
| - Yield Force at 5% strain MD | | mPa | 14.59 | 16.99 | 11.27 |
| - Yield Force at 5% strain TD | | mPa | 3.53 | 3.79 | N/A |
| - Tensile strenght at break MD | | mPa | 30.7 | 33.3 | 30.5 |
| - Tensile strenght at break TD | | mPa | 5.1 | 5.4 | N/A |
| - Elongation at break MD | | % | 89 | 81 | 162 |
| - Elongation at break TD | | % | 276 | 277 | N/A |
| - Energy MD | | $mJ/mm^3$ | 21 | 22 | 34 |
| - Energy TD | | $mJ/mm^3$ | 13 | 13 | N/A |
| - 1% Modulus MD | | mPa | 260 | 294 | 228 |
| - 1% Modulus TD | | mPa | 92 | 102 | N/A |
| Puncture resistance | ASTM D-5748 | | | | |
| - Max Compressive Load | | N | 8.16 | 8.76 | N/A |
| - Normalized Max Compressive Load | | N/μm | 0.51 | 0.52 | N/A |
| - Compressive extension at break | | mm | 57.36 | 58.44 | N/A |
| - Energy at break | | mJ | 2000 | 1900 | N/A |
| - Normalized Energy at break | | mJ/μm | 125.00 | 111.76 | N/A |
| Hydrohead Statstic Pressure | ASTM D-5748 | | | | |
| 1st drop-Avg | Based on WSP | mbar | 48.6 | 54.8 | N/A |
| 3rd drop-Avg | 80.6 | mbar | 49.2 | 55.3 | N/A |
| Water Vapor Transmission Rate | ASTM D-5748 | | | | |
| WVTR | Based on WSP 070.4.R3 at MOCON Lab | g/m2*24h | 19763 | 14826 | N/A |

## Example 2

### Formulation #1 - 20gsm

40% Exceed™ mPE 3527PA + 50% $CaCO_3$ + 7.5% ExxonMobil™ LDPE + 2.5% additive

### Formulation #2 - 20gsm

40% Enable™ mPE (density = 0.940 $g/cm^3$ and MI = 2.2 g/10 min (190°C, 2.16kg)) + 50% $CaCO_3$ + 7.5% ExxonMobil™ LDPE + 2.5% additive

Formulations 1 and 2 were first compounded into a free flowing pelletized material on a standard extrusion compounding line at standard processing conditions. The additives referenced above included white pigment, a thermal stabilizer, processing aids.

After this extrusion step, the compounds were extruded on a typical mono-layer cast line, characterized by the following features.

Screw size : 120 mm

Die width : 2.5 m

Die gap : 1.8 mm

Output: 200 kg/hr

After extrusion, the film was guided through an in-line heated MDO unit, stretching the initial film down to 20 gsm.

The following film properties were measured in Table 2.

**Table 2**

| Formulation | | Test Method | Formulation #1 | Formulation #2 |
|---|---|---|---|---|
| Tensile | | | | |
| Yield Strength (MPa) | MD | | 10.7 | 13.3 |
| | TD | | 3.8 | 5.1 |
| Elongation @ Yield (%) | MD | | 10.0 | 8.7 |
| | TD | ASTM D-822 | 6.4 | 5.6 |
| Tensile Strength (MPa) | MD | | 20.9 | 23.7 |
| | TD | | 5.6 | 6.0 |
| Elongation @ Break (%) | MD | | 199.0 | 185.0 |
| | TD | | 321.0 | 298.0 |
| Water Vapor Transmission Rate (WVTR) (g/m2*24h) | | Based on WSP 070.4.R3 at MOCON lab | 20,150 | 17,592 |
| Water Penetration Resistance (mmH20) | | ISO 0811 | 534 | 613 |

The data demonstrates that the stiffness of the backsheet as indicated primarily by yield strengths may be elevated by blending Enable™ mPE into the formulation. Without being bound to theory, it is believed that a proper level of long chain branching in Enable™ mPE provides the necessary overall relaxation time and results in more orientation for the film. Additionally, the mechanical properties of the inventive films provide for downgauging opportunities because a backsheet made with Enable™ mPE may be thinner but yet still provide suitable yield strengths correlating to good stiffness in the backsheet and overall hygiene article.

**Claims**

1. A hygiene article comprising:

   a) at least one top sheet;
   b) at least one absorbent core; and
   c) at least one backsheet;

   wherein the at least one backsheet comprises from 3 wt% to 60 wt% of a first polyethylene polymer, based upon the total weight of the at least one backsheet, and wherein the first polyethylene polymer has a melt index (MI) from 0.2 dg/min to 3.0 dg/min; a density from 0.918 $g/cm^3$ to 0.945 $g/cm^3$; and a melt index ratio (MIR) from 25 to 60, wherein the at least one backsheet comprises a second polyethylene polymer.

2. The hygiene article of claim 1, wherein the at least one backsheet comprises from 10 wt% to 40 wt% of the first polyethylene polymer.

3. The hygiene article of claim 1, wherein the at least one backsheet comprises from 15 wt% to 25 wt% of the first polyethylene polymer.

4. The hygiene article of any one of the preceding claims, wherein the first polyethylene polymer has a density from 0.920 $g/cm^3$ to 0.930 $g/cm^3$.

5. The hygiene article of any one of the preceding claims, wherein the first polyethylene polymer has a melt index (MI) from 0.3 dg/min to 1.0 dg/min.

6. The hygiene article of any one of the preceding claims, wherein the first polyethylene polymer has a melt index ratio (MIR) from 30 to 55.

7. The hygiene article of any one of the preceding claims, wherein the first polyethylene polymer has a melt index ratio (MIR) from 35 to 50.

8. The hygiene article of any one of the preceding claims, wherein the first polyethylene polymer has a melt strength of 0.01 cN to 1.0 cN.

9. The hygiene article of any one of the preceding claims, wherein the first polyethylene polymer has a melt strength of 0.02 cN to 0.1 cN.

10. The hygiene article of any one of the preceding claims, wherein the first polyethylene polymer has a g' branching index from 0.97 to 0.99.

11. A process to produce a hygiene article, the process comprising disposing the following layers, in order, unto one another to produce the hygiene article:

    a) at least one top sheet;
    b) at least one absorbent core; and
    c) at least one backsheet;

    wherein the at least one backsheet comprises from 3 wt% to 60 wt% of a first polyethylene polymer, based upon the total weight of the at least one backsheet, and wherein the first polyethylene polymer has a melt index (MI) from 0.2 dg/min to 3.0 dg/min; a density from 0.918 g/cm3 to 0.945 g/cm3; and a melt index ratio (MIR) from 25 to 60, wherein the at least one backsheet comprises a second polyethylene polymer.

12. The process of claim 11, further comprising orienting the at least one backsheet, optionally, in the machine direction and/or transverse direction.

13. The process of claim 11 or 12, wherein the at least one backsheet is cast film, optionally, a monolayer cast film or a multilayer cast film.

14. The process of claim 11 or 12, wherein the at least one backsheet is blown film, optionally, a monolayer blown film or a multilayer blown film.

15. The process of any one of claims 11-14, wherein the at least one backsheet comprises from 10 wt% to 40 wt% of the first polyethylene polymer.


**Patentansprüche**

1. Hygieneartikel, der

    a) mindestens eine Oberschicht,
    b) mindestens einen absorbierenden Kern und
    c) mindestens eine Rückschicht

    umfasst,
    wobei die mindestens eine Rückschicht 3 Gew.-% bis 60 Gew.-% eines ersten Polyethylenpolymers umfasst, bezogen auf das Gesamtgewicht der mindestens einen Rückschicht, und wobei das erste Polyethylenpolymer einen Schmelzindex (MI) von 0,2 dg/min bis 3,0 dg/min, eine Dichte von 0,918 g/cm$^3$ bis 0,945 g/cm$^3$ und ein Schmelzindexverhältnis (MIR) von 25 bis 60 aufweist, wobei die mindestens eine Rückschicht ein zweites Polyethylenpolymer umfasst.

2. Hygieneartikel nach Anspruch 1, bei dem die mindestens eine Rückschicht 10 Gew.-% bis 40 Gew.-% des ersten Polyethylenpolymers umfasst.

3. Hygieneartikel nach Anspruch 1, bei dem die mindestens eine Rückschicht 15 Gew.-% bis 25 Gew.-% des ersten Polyethylenpolymers umfasst.

4. Hygieneartikel nach einem der vorhergehenden Ansprüche, bei dem das erste Polyethylenpolymer eine Dichte von 0,920 g/cm$^3$ bis 0,930 g/cm$^3$ aufweist.

5. Hygieneartikel nach einem der vorhergehenden Ansprüche, bei dem das erste Polyethylenpolymer einen Schmelzindex (MI) von 0,3 dg/min bis 1,0 dg/min aufweist.

6. Hygieneartikel nach einem der vorhergehenden Ansprüche, bei dem das erste Polyethylenpolymer ein Schmelzindexverhältnis (MIR) von 30 bis 55 aufweist.

7. Hygieneartikel nach einem der vorhergehenden Ansprüche, bei dem das erste Polyethylenpolymer ein Schmelzindexverhältnis (MIR) von 35 bis 50 aufweist.

8. Hygieneartikel nach einem der vorhergehenden Ansprüche, bei dem das erste Polyethylenpolymer eine Schmelzfestigkeit von 0,01 cN bis 1,0 cN aufweist.

9. Hygieneartikel nach einem der vorhergehenden Ansprüche, bei dem das erste Polyethylenpolymer eine Schmelzfestigkeit von 0,02 cN bis 0,1 cN aufweist.

10. Hygieneartikel nach einem der vorhergehenden Ansprüche, bei dem das erste Polyethylenpolymer eine Verzweigungsindex g' von 0,97 bis 0,99 aufweist.

11. Verfahren zur Herstellung eines Hygieneartikels, bei dem die folgenden Schichten in Reihenfolge aufeinander abgelegt werden, um den Hygieneartikel herzustellen:

    a) mindestens eine Oberschicht,
    b) mindestens ein absorbierender Kern und
    c) mindestens eine Rückschicht,

    wobei die mindestens eine Rückschicht 3 Gew.-% bis 60 Gew.-% eines ersten Polyethylenpolymers umfasst, bezogen auf das Gesamtgewicht der mindestens einen Rückschicht, und wobei das erste Polyethylenpolymer einen Schmelzindex (MI) von 0,2 dg/min bis 3,0 dg/min, eine Dichte von 0,918 g/cm$^3$ bis 0,945 g/cm$^3$ und ein Schmelzindexverhältnis (MIR) von 25 bis 60 aufweist, wobei die mindestens eine Rückschicht ein zweites Polyethylenpolymer umfasst.

12. Verfahren nach Anspruch 11, bei dem ferner die mindestens eine Rückschicht gegebenenfalls in Längsrichtung und/oder Querrichtung orientiert wird.

13. Verfahren nach Anspruch 11 oder 12, bei dem die mindestens eine Rückschicht eine Gießfolie, gegebenenfalls eine Einschichtgießfolie oder eine Mehrschichtgießfolie ist.

14. Verfahren nach Anspruch 11 oder 12, bei dem die mindestens eine Rückschicht eine Blasfolie, gegebenenfalls eine Einschichtblasfolie oder eine Mehrschichtblasfolie ist.

15. Verfahren nach einem der Ansprüche 11 bis 14, bei dem die mindestens eine Rückschicht 10 Gew.-% bis 40 Gew.-% des ersten Polyethylenpolymers umfasst.

**Revendications**

1. Article d'hygiène comprenant :

    a) au moins une feuille supérieure ;
    b) au moins un noyau d'absorbant ; et
    c) au moins une feuille arrière ;

    dans lequel l'au moins une feuille arrière comprend de 3 % en poids à 60 % en poids d'un premier polymère de polyéthylène, sur la base du poids total de l'au moins une feuille arrière, et dans lequel le premier polymère de polyéthylène a un indice de fluidité à chaud (MI) de 0,2 dg/min à 3,0 dg/min ; une masse volumique de 0,918 g/cm$^3$

à 0,945 g/cm$^3$ ; et un rapport d'indice de fluidité à chaud (MIR) de 25 à 60, dans lequel l'au moins une feuille arrière comprend un deuxième polymère de polyéthylène.

2. Article d'hygiène selon la revendication 1, dans lequel l'au moins une feuille arrière comprend de 10 % en poids à 40 % en poids du premier polymère de polyéthylène.

3. Article d'hygiène selon la revendication 1, dans lequel l'au moins une feuille arrière comprend de 15 % en poids à 25 % en poids du premier polymère de polyéthylène.

4. Article d'hygiène selon l'une quelconque des revendications précédentes, dans lequel le premier polymère de polyéthylène a une masse volumique de 0,920 g/cm$^3$ à 0,930 g/cm$^3$.

5. Article d'hygiène selon l'une quelconque des revendications précédentes, dans lequel le premier polymère de polyéthylène a un indice de fluidité à chaud (MI) de 0,3 dg/min à 1,0 dg/min.

6. Article d'hygiène selon l'une quelconque des revendications précédentes, dans lequel le premier polymère de polyéthylène a un rapport d'indice de fluidité à chaud (MIR) de 30 à 55.

7. Article d'hygiène selon l'une quelconque des revendications précédentes, dans lequel le premier polymère de polyéthylène a un rapport d'indice de fluidité à chaud (MIR) de 35 à 50.

8. Article d'hygiène selon l'une quelconque des revendications précédentes, dans lequel le premier polymère de polyéthylène a une résistance à chaud de 0,01 cN à 1,0 cN.

9. Article d'hygiène selon l'une quelconque des revendications précédentes, dans lequel le premier polymère de polyéthylène a une résistance à chaud de 0,02 cN à 0,1 cN.

10. Article d'hygiène selon l'une quelconque des revendications précédentes, dans lequel le premier polymère de polyéthylène a un indice de ramification g' de 0,97 à 0,99.

11. Procédé de production d'un article d'hygiène, le procédé comprenant la disposition des couches suivantes, dans l'ordre, les unes au-dessus des autres, pour produire l'article d'hygiène :

   a) au moins une feuille supérieure ;
   b) au moins un noyau d'absorbant ; et
   c) au moins une feuille arrière ;

dans lequel l'au moins une feuille arrière comprend de 3 % en poids à 60 % en poids d'un premier polymère de polyéthylène, sur la base du poids total de l'au moins une feuille arrière, et dans lequel le premier polymère de polyéthylène a un indice de fluidité à chaud (MI) de 0,2 dg/min à 3,0 dg/min ; une masse volumique de 0,918 g/cm$^3$ à 0,945 g/cm$^3$ ; et un rapport d'indice de fluidité à chaud (MIR) de 25 à 60, dans lequel l'au moins une feuille arrière comprend un deuxième polymère de polyéthylène.

12. Procédé selon la revendication 11, comprenant en outre l'orientation de l'au moins une feuille arrière, facultativement, dans la direction machine et/ou la direction transversale.

13. Procédé selon la revendication 11 ou 12, dans lequel l'au moins une feuille arrière est un film coulé, facultativement, un film coulé monocouche ou un film coulé multicouche.

14. Procédé selon la revendication 11 ou 12, dans lequel l'au moins une feuille arrière est un film soufflé, facultativement, un film soufflé monocouche ou un film soufflé multicouche.

15. Procédé selon l'une quelconque des revendications 11 à 14, dans lequel l'au moins une feuille arrière comprend de 10 % en poids à 40 % en poids du premier polymère de polyéthylène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4341216 A
- US 5171239 A
- US 5928209 A
- US 7501357 B
- US 20130295364 A
- US 20100062231 A
- WO 0151546 A
- WO 2014123797 A
- WO 2012058789 A
- WO 2002007662 A
- WO 1991019471 A

**Non-patent literature cited in the description**

- **RANDALL.** *Journal of Macromolecular Science, Rev. Macromol. Chem. Phys.,* vol. C29 (2&3), 285-297
- *Macromolecules,* 2000, vol. 33, 7489-7499
- **SCHOLTE et al.** *J. App. Polymer Sci,* 1984, vol. 29, 3763-3782
- Polypropylene Handbook. Carl Hanser Verlag, 1996